(19)

Europäisches
Patentamt
European
Patent Office
Office européen
des brevets

(11) **EP 3 207 873 A1**

(12) **EUROPEAN PATENT APPLICATION**

(43) Date of publication:
   **23.08.2017 Bulletin 2017/34**

(51) Int Cl.:
   **A61B 6/00** (2006.01)      **A61B 5/20** (2006.01)
   **A61B 5/00** (2006.01)

(21) Application number: **17164592.2**

(22) Date of filing: **15.02.2013**

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**

(30) Priority: **17.02.2012 US 201261600182 P**

(62) Document number(s) of the earlier application(s) in
   accordance with Art. 76 EPC:
   **13749013.2 / 2 814 393**

(71) Applicant: **Pharmacophotonics, Inc.**
   **Indianapolis, IN 46202 (US)**

(72) Inventors:
   • **MEIER, Daniel**
     **Indianapolis, IN 46234 (US)**

   • **MOLITORIS, Bruce**
     **Indianapolis, IN 46260 (US)**
   • **REILLY, Erinn**
     **Indianapolis, IN 46229 (US)**
   • **SANDOVAL, Ruben**
     **Indianapolis, IN 46220 (US)**

(74) Representative: **Atkins, James Gordon John et al**
   **Kilburn & Strode LLP**
   **20 Red Lion Street**
   **London WC1R 4PJ (GB)**

Remarks:
   This application was filed on 03.04.2017 as a
   divisional application to the application mentioned
   under INID code 62.

(54) **ORAL PROBE FOR MEASURING FLUORESCENT INTENSITY**

(57)    Methods of measurement of biometric indicators in a mammalian subject are described. Biometric indicators of interest include hematocrit, plasma volume, volume of distribution, and glomerular filtration rate. The methods are especially applicable to subjects with rapid blood loss and to subjects with unstable hematocrits. Hematocrit may be measured by administering an injectate with a dynamic fluorescent marker and a static fluorescent marker, or a single static marker with two fluorescent tags, into the vascular system of the subject, and monitoring the emission intensities of the markers or fluorescent tags over a period of time. Hematocrit may then be calculated using a calibrated spectrometric analyzer by determining the raw ratio of the markers at To, calculating the apparent hematocrit, and applying a correction factor.

EP 3 207 873 A1

# Description

## CROSS-REFERENCE TO RELATED APPLICATIONS

This application claims priority from U.S. Provisional Application No. 61/600,182, filed February 17, 2012, the disclosure of which is incorporated herein in its entirety.

## BACKGROUND OF THE INVENTION

The present invention relates generally to compositions and methods for collecting biometric information from a mammalian subject, and preferably a human subject. More particularly, the present invention is directed to fluorescent spectrometric methods for quantifying hematocrit and other physiological parameters of a subject by introducing a calibrated injectate comprising one or more fluorescent markers into the vascular system of the subject, and monitoring the emission intensities of the fluorescent marker(s) over a period of time.

Biometric indicators are valuable tools used by medical practitioners to aid in the diagnosis of a patient, and their ability to determine the proper course of medical treatment is often limited by access to rapid and accurate quantitative biometric information. Some common biometric indicators used by medical practitioners include core body temperature, blood pressure, heart and respiratory rates, blood oxygenation and hematocrit, glomerular filtration rate ("GFR"), and the like. While a medical practitioner may prefer to assess multiple biometric indicators prior to deciding on a particular treatment, the patient's condition may deteriorate faster than the indicators may be assessed. In these situations, medical practitioners are required to make decisions with limited information, potentially decreasing a patient's chance of survival. Therefore, there is a need for methods and devices capable of quickly and accurately determining one or multiple biometric indicators.

Hematocrit ("HCT"), also commonly referred to as packed cell volume, is the volumetric proportion of red blood cells to total blood volume. Hematocrit may vary based on gender and age. Typical hematocrit levels for healthy adult humans are about 45% for males and about 40% for females. HCT is commonly used in the early stages of patient care to aid in diagnosis, and abnormal HCT levels may indicate certain medical conditions. Abnormally high hematocrit levels have been associated with dengue fever, hypoxia related diseases such as COPD, dehydration, and the like, while abnormally low hematocrit levels have been associated with hemorrhaging, chronic kidney disease resulting from low erythropoietin levels, decongestive heart failure and the like.

There are several methods and devices currently available for determining HCT. The name "packed cell volume" is derived from the traditional method of determining HCT where centrifugal forces are applied to a heparinized sample of blood, followed by the measurement of the volumetric proportion of red blood cells to total blood volume. While this method is relatively accurate, the blood sample is often sent to a medical laboratory separate from the patient care room for analysis, which may drastically increase the sample processing time and limits its utility in time sensitive medical situations.

Noninvasive spectrometric HCT devices, such as the Critxcan" by Hema Metrics (Kaysville, UT), have been developed to address some of the limitations of traditional methods by taking advantage of the optical properties of blood. Blood is known in the art to have nonlinear optical properties, which results in wavelength-dependent optical attenuation coefficients. These attenuation coefficients are also known to be hematocrit dependent. The attenuation coefficients of some wavelengths, such as 620 and 680 nm, are known to be relatively linear, while other wavelengths, such as 488 and 594 nm, are known to be nonlinear. Noninvasive spectrometric HCT devices measure the relative attenuation of two or more optical signals with different wavelengths and attenuation coefficients across a highly vascularized portion of tissue. Conventional operation begins by transmitting an optical signal through a first optical conduit to a first optical interface and into portion of tissue, where the tissue optically interacts with the optical signal producing an attenuated signal. The attenuated signal may then either be transmitted or reflected to a second optical interface where a second optical conduit transmits the optical information to a detector. A ratio of the attenuated signal strength is then transformed into a hematocrit level using predetermined mathematical relationships. These noninvasive spectrometric HCT methods and devices typically use one or multiple conventional noise processing techniques, such as relative pulsatile signal analysis, in order to achieve a sufficient signal-to-noise ratio to permit accurate measurements.

An "optical conduit", as used in the present application, means a transparent optical waveguide, such as a fiber optic cable or an optically reflective pipe, which is capable of transmitting optical signals from one location to another. An optical conduit may comprise of optical waveguide, such as a single fiber optic cable, or multiple optical waveguides arranged about a common optical source and optical interface, such as a bundle of fiber optic cables. An "optical interface" is the optical boundary that separates the terminal end of an optical conduit distal to an optical source or optical signal detector from an external environment.

While noninvasive spectrometric HCT devices and other noninvasive direct spectrometric devices, such as pulse oximeters, provide nearly instantaneous and relatively accurate results, they are limited by their need for a relatively large portion of tissue to accommodate the multiple optical interfaces. These devices are also limited by the ability of the skin tissue to transmit sufficient levels of optical information, and their need for a fixed optical geometry between the multiple optical interfaces, often resulting in mechanically rigid devices. The fixed optical geometry between multiple interfaces further limits the ability for these devices to maintain a sterile environment through the use of disposable sterile barriers, such as low-density polyethylene (LDPE) plastic liners, due to the optical noise resulting from light scattering at the multiple optical interfaces. As a result, the spectrometric sensors themselves are often disposed of to maintain a sterile environment. Disposable pulse oximeters used in intensive care units are a common example. While this strategy maintains a sterile environment, it also significantly increases the costs of medical care compared to the use of traditional sterile barriers.

Invasive indirect fluorescent spectrometric methods and devices for monitoring glomerular filtration rate ("GFR") and determining the plasma volume through a single flexible optical conduit have been previously disclosed in United States Patent Application Nos. 12/425,827 and 12/946,471, and PCT Patent Application Nos. PCTIUS2009/040994 and PCT/US 10/32997. As defined in the present application, the term "plasma volume" refers to the total amount of plasma contained in the vasculature of a subject, while the term "circulating plasma volume" refers to the amount of flowing plasma contained in the vasculature of the subject. Although the measurements for "plasma volume" and "circulating plasma volume" are similar and related, they are not the same.

Fluorescent spectrometric systems conventionally include a light source for exciting a fluorescent marker, an optical conduit for transmitting the light source and receiving the fluorescent signal, a light detector for measuring the fluorescent signal, a means for storing the spectrometric data set, such as a digital memory storage device, a means for processing the spectrometric data set to calculate GFR, such as a digital computation processor, and an output for the calculated GFR, such as a digital display.

GFR may be determined using a fluorescent spectrometric system by inserting the terminal end of the optical conduit into the blood vessel of an animal subject, administering a bolus injection containing a dynamic marker of a first set of fluorescent characteristics (i.e., excitation and emission wavelengths) and steady state marker of a second set of fluorescent characteristics, fluorescently monitoring the decrease of the dynamic marker relative to the static marker over a period of time, and calculating GFR based on relative change in markers using a series of mathematical steps. Alternatively, the volume of distribution, as defined earlier, may be determined by measuring the fluorescent signal strength of a static marker once it has reached a quasi-stable vascular distribution, and correlating the decrease in signal strength to the dilution of the bolus injection.

Fluorescent spectrometric systems typically use minimally invasive optical conduits to monitor the fluorescent markers. While it would be advantageous to use a noninvasive optical conduit to monitor fluorescent markers, relatively low signal intensities combined with tissue auto-fluorescence have inhibited their use. The oral cavity is a unique highly vascularized location that may limit tissue auto-fluorescence and provide potentially high signal-to-noise ratio due to the relatively thin tissue; however, the oral cavity is also a location of continual movement and readjustment. All optical oral probes currently available require continuous manual positioning by a well-trained operator to overcome oral cavity movement.

Conventional spectrometric methods of determining HCT assume that blood has constant optical properties during the observation period. This assumption is directly used to correlate attenuation of optical signals of multiple wavelengths to HCT independent of the length of the observation period. While conventional fluorescent injectates used to determine GFR and plasma volume are being developed for human use and have shown favorable biocompatibility, and HCT is often assessed with GFR and plasma volume in certain medical situations, they have not been used to measure HCT due to the dynamic optical properties resulting from the constantly changing concentrations of the dynamic marker used in the injectate.

Noninvasive direct spectrometric methods and devices require the use of multiple optical interfaces and optical conduits at a fixed geometry, resulting in devices that are mechanically rigid and difficult to sterilize. While fluorescent spectrometric systems are able to measure GFR and plasma volume via a single optical conduit, they are conventionally unable to measure hematocrit due to the constantly changing concentrations of the dynamic markers. Thus, there remains a clinical need to develop a sterile and accurate method of fluorescently measuring the hematocrit of a patient. The present invention is provided to solve the problems discussed above and other problems, and to provide advantages and aspects not provided by prior diagnostic techniques. A full discussion of the features and advantages of the present invention is deferred to the following detailed description, which proceeds with reference to the accompanying drawings.

## SUMMARY OF THE INVENTION

The present invention relates to systems and methods of measuring hematocrit, GFR and other biometric indicators in a mammalian subject.

An aspect of the present invention is to provide an injectate for measuring hematocrit in a mammalian subject, the injectate may comprise (a) a first fluorescent marker having a first excitation wavelength and a first emission wavelength; (b) a second fluorescent marker having a second excitation wavelength and a second emission wavelength; and (c) an injectate carrier; wherein the first fluorescent marker is a dynamic molecule, and the second fluorescent marker is a static molecule; and wherein the injectate has undergone calibration to provide a calibration identification containing parameters of the injectate.

In another aspect, the injectate may comprise (a) a single static marker having a first fluorescent tag and a second fluorescent tag, wherein the first fluorescent tag has a first excitation wavelength and a first emission wavelength and the second fluorescent tag has a second excitation wavelength and a second emission wavelength; and (b) an injectate carrier; wherein the injectate has undergone calibration to provide a calibration identification containing parameters of the injectate.

A further aspect of the present invention is to provide a method for measuring hematocrit of a mammalian subject which may comprise the following steps: (a) providing an injectate comprising (i) a first fluorescent marker having a first excitation wavelength and a first emission wavelength; (ii) a second fluorescent marker having a second excitation wavelength and a second emission wavelength; and (iii) an injectate carrier; wherein the first fluorescent marker is a dynamic molecule and the second fluorescent marker is a static molecule; (b) calibrating the injectate to obtain a calibration identification of the injectate containing parameters of the injectate; (c) inputting the parameters of the calibration identification of the injectate to a fluorescent detector to calibrate the fluorescent detector; (d) obtaining a species specific hematocrit curve; (e) introducing the injectate into vascular system of the mammalian subject; (f) exciting the first fluorescent marker with a first excitation wavelength and exciting the second fluorescent marker with a second excitation wavelength at an optical interface of an optical probe and the vascular system, or a sample taken from the vascular system; (g) measuring the first emission intensity of the first emission wavelength of the first fluorescent marker and measuring the second intensity of the second emission wavelength of the second fluorescent marker at the optical interface of the optical probe and the vascular system, or a sample taken from the vascular system, using the calibrated fluorescent detector to obtain a spectrometric data set comprising a first emission fluorescent intensity curve from the first fluorescent marker and a second emission fluorescent intensity curve from the second fluorescent marker; (h) obtaining a raw ratio (as defined herein) from the spectrometric data set to determine an apparent hematocrit of the subject from the species specific hematocrit curve; (i) obtaining a correction factor for determining the hematocrit of the mammalian subject; and (j) applying the correction factor to the apparent hematocrit to determine the hematocrit of the mammalian subject.

In a still further aspect of the invention, a method for measuring the hematocrit of a mammalian subject may comprise the following steps: (a) providing an injectate comprising (i) a single static marker having a first fluorescent tag and a second fluorescent tag wherein the first fluorescent tag has a first excitation wavelength and a first emission wavelength and the second fluorescent tag has a second excitation wavelength and a second emission wavelength; and (ii) an injectate carrier; (b) calibrating the injectate to obtain a calibration identification of the injectate containing parameters of the injectate; (c) inputting the parameters of the calibration identification of the injectate to a fluorescent detector to calibrate the fluorescent detector; (d) obtaining a species specific hematocrit curve; (e) introducing the injectate into the vascular system of the mammalian subject; (f) exciting the first fluorescent tag with a first excitation wavelength and exciting the second fluorescent tag with a second excitation wavelength at an optical interface of an optical probe and the vascular system, or a sample taken from the vascular system; (g) measuring the first emission intensity of the first emission wavelength of the first fluorescent tag and measuring the second intensity of the second emission wavelength of the second fluorescent tag at the optical interface of the optical probe and the vascular system, or a sample taken from the vascular system, using the calibrated fluorescent detector to obtain a spectrometric data set comprising a first emission fluorescent intensity curve from the first fluorescent tag and a second emission fluorescent intensity curve from the second fluorescent marker; (h) obtaining a raw ratio (as defined herein) from the spectrometric data set to determine an apparent hematocrit of the subject from the species specific hematocrit curve; (i) obtaining a correction factor for determining the hematocrit of the mammalian subject; and (j) applying the correction factor to the apparent hematocrit to determine the hematocrit of the mammalian subject.

A yet still further aspect of the invention is to provide a periodic blood sampling technique for use in measuring hematocrit, GFR and other biometric indicators. This technique may utilize a dynamic and static marker as described above, and avoids the necessity for continuous sampling form the subject's blood stream, and utilizes blood samples taken at variable times ranging from 10 to 60 minute intervals. It has been found that the $T_0$ concentration of the dynamic marker, as defined herein, can be measured directly using the concentration of the static marker, also as defined herein, since the ratio of the concentration of the two markers is known at the time of injection. The concentration of the dynamic marker at $T_0$ can be calculated by using the intensity of the static marker to determine its concentration once mixing in the flowing plasma is completed, normally within 10 to 15 minutes following injection. This allows the use of fewer blood samples to determine GFR over a shorter time span, usually within 2 hours or less. The use of the static marker allows the $T_0$ concentration of the dynamic marker to be predicted, thus providing a measurement of the dynamic marker that cannot be directly measured.

Yet another aspect of the present invention is to provide an oral probe for use within the oral cavity of a mammalian subject for measuring fluorescent intensity of a fluorescent molecule in the vascular system of the mammalian subject comprising: (a) a longitudinal optical conduit having a proximal end and a distal end; (b) an optical interface at the distal end of the optical conduit;

and (c) an oral stabilizing guide to limit the optical conduit within the oral cavity; wherein the fluorescent intensity of a fluorescent molecule in the vascular system is transmitted from the vascular system to the optical conduit through the optical interface at the distal end of the optical conduit to the proximal end of the optical conduit.

The present invention may be used in combination with previous methods and devices to achieve simultaneous measurement of blood volume, plasma volume, volume of distribution, glomerular filtration rate (GFR),and hematocrit from a single injection of the injectate.

Other features and advantages of the present novel technology will be apparent from the following description.

## BRIEF DESCRIPTION OF THE DRAWINGS

To understand the present invention, it will now be described by way of example, with reference to the accompanying drawings in which:

FIG. 1 is an example of the results of a step dose blood test set.

FIG. 2 is a plot of each VFI component (intercept forced to zero), using the average signal level and amount of each component at each dose step.

FIG. 3 is a plot of fluorescence intensity level vs. HCT.

FIG. 4 is plot of the HCT data of FIG. 3 taking the ratio of the signal levels of Component 1 to Component 2, and plotting that ratio versus the HCT calculated at each stage.

FIG. 5 is an example of a spectrometric data set obtained from administering and fluorescently monitoring of the vascular distribution of an injectate of the present invention.

FIG. 6 is an example of a calibration curve of the fluorescence intensity signal level vs. material amount;

FIG. 7 is an example of a calibration curve of the fluorescence intensity signal level vs. HCT.

FIG. 8 is an example of a calibration curve of the raw ratio (concentration ratio of the dynamic and static markers at $T_0$) of the fluorescent markers vs. HCT.

FIG. 9 is an example of a calibration curve of fluorescent intensity signal level vs. HCT using a single static marker with two fluorescent tags.

## DETAILED DESCRIPTION

For the purposes of promoting an understanding of the principles of the invention, reference will be made to the embodiments illustrated in the drawings and specific language will be used to describe the same. It will nevertheless be understood that no limitation of the scope of the invention, with such alterations and further modifications in the illustrated device and such further applications of the principles of the technology as illustrated therein being contemplated as would normally occur to one skilled in the art to which the technology relates.

The present invention generally relates to compositions and methods for the measurement of biometric indicators in a mammalian subject. The mammalian subject may be a human. The biometric indicators of interest include, but are not limited to, hematocrit, blood volume, plasma volume, volume of distribution, and glomerular filtration rate (GFR). The present invention may be especially applicable to subjects having rapid blood loss without determining the hematocrits and subjects with unstable hematocrits.

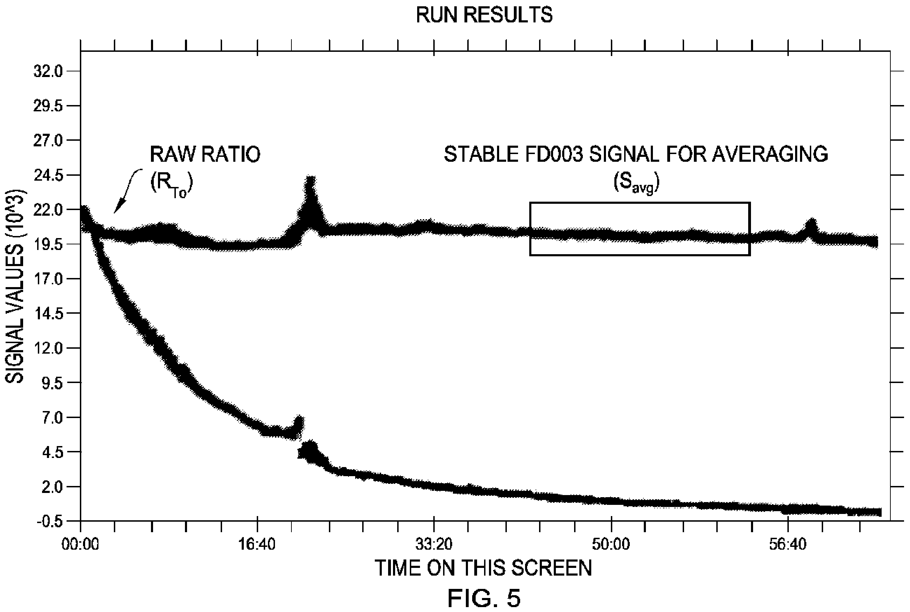

In brief, hematocrit may be determined by analyzing a spectrometric data set, as shown in FIG. 5, obtained from the administration and fluorescent monitoring of the vascular distribution of an injectate of the present invention for a period of time that includes the peak vascular distribution of the markers at $T_0$. A calibrated spectrometric analyzer of the present application may be used to determine HCT from the spectrometric data set. One advantage of the present invention is the ability to utilize dynamic and static markers to determine HCT in a subject.

A spectrometric data set as used in the present application means a data set resulting from the administration and fluorescent monitoring of the vascular distribution of an injectate containing two or more fluorescent markers of different fluorescent wavelengths, where one of the fluorescent markers is a dynamic marker and on of the fluorescent markers is a static marker, for a period of time that includes the peak vascular distribution of the fluorescent markers.

A calibrated spectrometric analyzer of the present invention ("Calibrated Spectrometric Analyzer") comprises an input for a spectrometric data set, an input for calibration identification, a computational engine for calculating hematocrit, and an output for reporting a calculated hematocrit. The calibration identification may be set with factory predicted average injectate parameters during manufacturing and stored in a computationally accessible location, it may be updated indirectly via a change in software or hardware, or may be updated directly by uploading injectate specific parameters. Injectate specific parameters may be input through the use of a manual device, such as a keypad or touch screen, through the use of a semi-automated device, such as a barcode scanner, or through the use of an indirect automated process, such as by the use of a wireless software update.

### Injectate for Determining Hematocrit

The injectate of the present application comprises a first fluorescent marker, a second fluorescent marker, and an injectate carrier. Each fluorescent marker has its own distinct fluorescent characteristics, i.e. distinct excitation wavelengths and emission wavelengths.

The first fluorescent marker has a first excitation wavelength and a first emission wavelength. The second fluorescent marker has a second excitation wavelength and a second emission wavelength. A fluorescent marker is any molecule containing a fluorophore which causes the molecule to be fluorescent. Many known fluorescent dyes can serve as fluorescent markers of the present invention, such as but are limited to rhodamine dyes or its derivatives (e.g., Texas Red®), fluorescein or its derivatives (e.g. fluorscein isothiocyante (FITC)), coumarin and cyanine. The fluorescent dye may be associated, for example via conjugation, with another macromolecule to provide an intended molecular weight for the fluorescent dye. Examples of macromolecules include but are not limited to polymers, proteins, dextrans, cell uloses, carbohydrates and nucleic acids. The macromoleulses can be naturally occurring compounds, or synthetic compounds. Methods for conjugating macromolecules with fluorescent dyes are well known in the art.

The first fluorescent marker is a dynamic molecule and the second fluorescent marker is a static molecule.

A "dynamic molecule" is a molecule of sufficiently low molecular mass to permeate the blood vessel walls or the vasculature of a subject. Dynamic molecules are known in the art to have a molecular mass less than 50 kDa, and more typically have a molecular mass less than 20 kDa.

A "static molecule" is a molecule of sufficiently high molecular mass to significantly limit its blood vessel wall permeability. Static markers may reach a quasi-stable vascular concentration for a period of time, although such markers may ultimately be cleared from the vasculature. Static markers are known in the art to have a molecular mass greater than 50 kDa, and more typically have a molecular mass greater than 200 kDa. Such markers can remain in the vasculature for a time period of between about 1 or 2 hours, to 12 hours or longer, depending on the molecular mass of the marker as well as other factors.

In an embodiment, the injectate may comprise a static marker having two fluorescent tags attached to the same static marker and an injectate carrier. Each fluorescent tag has its own distinct fluorescent characteristics, i.e. distinct excitation wavelengths and emission wavelengths. An example of such a static marker is a macromolecule, such as dextran with molecular mass greater than 50 kDa, conjugated with two different fluorescent dyes, such as Texas Red® and fluorescein or its derivatives.

The fluorescent markers are not metabolized within the subject during the period of time of measuring the biometric indicators. What is meant by "not metabolized within the subject" in the present application is that the marker has a half life ($T_{1/2}$,) of 4 hours or greater in the vascular system of the subject.

In the present invention, the two injectates mentioned previously can be used interchangeably. It is not important whether the injectate has two separate fluorescent markers providing two distinct fluorescent characteristics, or the injectate has only one marker having two fluorescent tags providing two distinct fluorescent characteristics are present. What is important is that the injectate provides two distinct fluorescent emission signals in order to allow the measurement of the biometric indicators as described in the present application. Thus, when reference is made to using an injectate having two fluorescent markers in the present application, this is also intended to include and refer to an injectate having only one marker but with two fluorescent tags on the molecule. The subsequent steps leading to the measuring of the hematocrit and other biometric indicators are otherwise identical. However, since the injectate comprising only one molecule uses a static marker without a dynamic marker, the injectate can be used to measure the hematocrit and other biometric indicators, but not GFR which requires at least two markers.

The term "injectate carrier" as used in the present application means a biologically acceptable fluid capable of solubilizing and delivering the fluorescent markers to aid in the delivery and biocompatibility of the fluorescent markers. Examples of suitable carriers include but are not limited to buffers, saline and the like.

Calibration Identification and Calibration Identifier

The injectate of the present invention is calibrated to provide a calibration identification containing parameters of the injectate.

A term "calibration identification" as used in the present invention means a collection of fluorescent injectate parameters used in the calculation of HCT from a spectrometric data set. The parameters may comprise the Visible Fluorescence Injectate (VFI) lot number and calibrated fluorescent intensity of each fluorescent marker or each fluorescent tag on the same marker.

A calibration identification can be represented as a calibration identifier represented by a series of numbers or signals. In an embodiment, the series of numbers or signals may be an optical machine-readable representation of data, such as but not limited to bar codes. Algorithms to convert the calibration identifier to a bar code calibration identifier to well known to those in the art.

The calibration identification may be set with factory predicted average injectate parameters during manufacturing, and stored in a computationally accessible location. It may be updated indirectly via a change in software or hardware, or may be updated directly by uploading injectate specific parameters.

Injectate specific parameters contained in the calibration identification may be input into another device, such as a fluorescent detector or a spectrometric analyzer, through the use of a manual device, such as a keypad or touch screen, through the use of a semiautomated device, such as a barcode scanner, or through the use of an indirect automated process, such as a wireless software update.

The reference standard fluorescent intensities used to generate the calibration curves used in calculating the biometric parameters may be represented in the calibration identifier as set value 1000 with a letter designation for each fluorescent marker of different fluorescent wavelength immediately following (i.e., 1000a; 1000b); fluorescent intensity variance from the reference standard for each fluorescent marker may be represented in the calibration identifier as a representative equivalent increase or decrease to the set value of 1000.

A sample calibration identifier is shown below:

LOTIOIAI034B0975

Information contained:

VFI Lot No.: 101
Fluorescent Marker 1 (A) intensity from calibration: 1034
Fluorescent Marker 2 (B) intensity from calibration: 0975

Calibrated Injectate

A calibrated injectate of the present invention ("Calibrated Injectate") may comprise a first fluorescent marker or fluorescent tag having a first hematocrit dependent fluorescent attenuation coefficient, a second fluorescent marker or fluorescent tag having a second hematocrit dependent fluorescent attenuation coefficient, an injectate carrier, and a calibration identification. The calibration identification may be provided separately from the Calibrated Injectate, may be provided with the Calibrated Injectate, or may be provided as a Calibration Identification. A Calibrated Injectate may be used to further improve the accuracy and precision of a Calibrated Spectrometric Analyzer by correcting for the optical batch variance resulting from the multiple manufacturing steps.

A calibration method of the present invention used to produce a Calibrated Injectate comprises a set of fluorescent intensity standards for each fluorescent marker or fluorescent tag, a set preparation procedure for creating working standard solutions and calibration solution for calibrating a fluorescence detector, and a fluorescence detector used to read the fluorescent intensity of each fluorescent marker in calibrations solution and injectate. From fluorescent marker standard solutions the set procedure is followed to create a working standard solution and a calibration solution. The calibration solution is used in the same fluorescent intensity range for each marker as the injectate. The calibration solution is used to set the parameters of the fluorescence detector. Then using the same set procedure, a test solution is made using the injectate to be calibrated. Using the calibrated fluorescence detector, the injectate test solution for the calibration identification for a Calibrated Injectate are generated.

A Calibrated Spectrometric Analyzer

A calibrated spectrometric analyzer of the present invention ("Calibrated Spectrometric Analyzer") comprises an input for a spectrometric data set, an input for a calibration identifier, a computational engine for calculating hematocrit, and an output for reporting a calculated hematocrit.

Method for Determining Hematocrit

In brief, hematocrit may be determined by analyzing a spectrometric data set obtained from administering and fluorescently monitoring of the vascular distribution of an injectate containing two or more fluorescent markers of different fluorescent wavelengths, where at least one of the fluorescent markers is a dynamic marker, for a period of time that includes the peak vascular distribution of the markers. Alternatively, the injectate may contain only one static marker having two fluorescent tags on the marker. A novel calibrated spectrometric analyzer of the present application may be used to determine HCT from a spectrometric data set. One advantage of the present invention is its ability to utilize dynamic markers to determine HCT in an animal subject.

The term "time zero" or "$T_0$", as used herein, is the moment in a spectrometric data set that is characterized by the peak fluorescent signal intensity of intravenously injected fluorescent markers. $T_0$ typically occurs within the first minute following bolus intravenous injection. $T_0$ is used to signify the start of the biometric parameter fluorescent signal analysis. The term "raw ratio" as used in the present application may be defined as the ratio of fluorescent signal intensities at $T_0$, i.e. the ratio of the dynamic marker ("small marker", indicating a smaller molecular weight, or "green marker", indicating a fluorescent tag emitting in the green spectrum) to the static marker ("larger marker", indicating a larger molecular weight, or a "red marker", indicating a fluorescent tag emitting in the red spectrum). An important aspect of the present technology is the use of the raw ratio to determine HCT, GFR, and other biometric parameters in an optically dynamic environment.

It has been found that up to 1/2 of the small marker is filtered from the blood stream after only about 15 minutes following the initial bolus infusion of a dynamic marker and a static marker. Accordingly, following the procedure of the present invention, the concentration of the dynamic marker at $T_0$ can be accurately predicted using, for instance, a spectrometric analyzer to measure the concentration of the static marker at 10 to 15 minute intervals as described herein. This is a significant advance since it permits the use of periodic biometric sampling, i.e. sampling the vasculature every 10 to 60 minutes, as contrasted to a continuous sampling procedure, and the total test time can be shortened to about 1 to 2 hours in duration from about 6 hours currently.

The raw ratio calculated in the present application may be used, in turn, to calculate the hematocrit observed at the optical interface of an optical probe, referred to in the present application as the apparent HCT. The apparent hematocrit obtained from invasive probes may be different from a subject's true HCT. This may be attributed to fluid dynamic anomalies occurring near an optical interface inserted in a flowing system. True HCT may be calculated from apparent HCT by applying a correction factor. A correction factor may be in the range of 1 to 10 percent of HCT, and more specifically in the range of 4-5 percent of HCT. A typical calculation of the correction factor is shown in the Examples herein.

A method for determining a species specific HCT curve comprises the following components: a calibrated fluorescence detector, a Calibrated Injectate, and a test volume of species specific blood. A procedure is performed to maintain a constant total test volume and constant concentration of Calibrated Injectate in the test volume while altering the HCT in the test volume. A calibrated fluorescence detector is set up and configured to read the fluorescence intensities of the test volume throughout the procedure. A test volume is prepared, with a known HCT ($H_{calib}$), as determined by conventional methods, and a measured total volume ($V_t$). A known volume of Calibrated Injectate is added to the test volume. A separate volume is created from normal saline and Calibrated Injectate, with an equivalent concentration of Calibrated Injectate added to the test volume. This solution is used to replace removed volume from the test volume during the procedure. A series of repetitive steps are then used to create different HCT levels in the test volume. A volume (x) is removed from the test volume, discarded, and replaced with an equivalent volume (x) of prepared saline solution. The system is allowed to stabilize, and the HCT is calculated at each stage based on the dilution of HCT. The average signal level of a "flat portion" of data at each HCT level tested is determined as shown, where $V_t$ is the total volume in the test set, $V_e$ is the volume exchanged (blood for saline), $H^0$ is the starting HCT (prior to volume exchange) and H' is the new HCT (post volume exchange). A hematocrit dependent curve is produced where the raw ratio is an input and the apparent hematocrit is the output.

Thus, the method for measuring hematocrit of a mammalian subject may comprise the following steps: (a) providing an injectate comprising (i) a first fluorescent marker having a first excitation wavelength and a first emission wavelength; (ii) a second fluorescent marker having a second excitation wavelength and a second emission wavelength; and (iii) an injectate carrier; wherein the first fluorescent marker is a dynamic molecule and the second fluorescent marker is a static molecule; (b) calibrating the injectate to obtain a calibration identification of the injectate containing parameters of the injectate; (c) inputting the parameters of the calibration identification of the injectate to a fluorescent detector to calibrate the fluorescent detector; (d) obtaining a species specific hematocrit curve; (e) introducing the injectate into vascular system of the mammalian subject; (f) exciting the first fluorescent marker with a first excitation wavelength and exciting the second fluorescent marker with a second excitation wavelength at an optical interface of an optical probe and the vascular system, or a sample taken from the vascular system; (g) measuring the first emission intensity of the first emission wavelength of the first fluorescent marker and measuring the second intensity of the second emission wavelength of the second fluorescent marker at the optical interface of the optical probe and the vascular system, or a sample taken from the vascular system, using the calibrated fluorescent detector to obtain a spectrometric data set comprising a first emission fluorescent intensity curve from the first fluorescent marker and a second emission fluorescent intensity curve from the second fluorescent marker; (h) obtaining a raw ratio from the spectrometric data set to determine an apparent hematocrit of the subject from the species specific hematocrit curve; (i) obtaining a correction factor for determining the hematocrit of the mammalian subject; and (j) applying the correction factor to the apparent hematocrit to determine the hematocrit of the mammalian subject. Other biometric indicators such as blood volume, volume of distribution and glomerular filtration rate, can also be determined using this technique.

In an embodiment, the method for measuring hematocrit of a mammalian subject may comprise the following steps: (a) providing an injectate comprising (i) a static marker having a first fluorescent tag and a second fluorescent tag wherein the first fluorescent tag has a first excitation wavelength and a first emission wavelength; and the second fluorescent tag has a second excitation wavelength and a second emission wavelength; and (ii) an injectate carrier; (b) calibrating the injectate to obtain a calibration identification of the injectate containing parameters of the injectate; (c) inputting the parameters of the calibration identification of the injectate to a fluorescent detector to calibrate the fluorescent detector; (d) obtaining a species specific hematocrit curve; (e) introducing the injectate into vascular system of the mammalian subject; (f) exciting the first fluorescent tag with a first excitation wavelength and exciting the second fluorescent tag with a second excitation wavelength at an optical interface of an optical probe and the vascular system, or a sample taken from the vascular system; (g) measuring the first emission intensity of the first emission wavelength of the first fluorescent tag and measuring the second intensity of the second emission wavelength of the second fluorescent tag at the optical interface of the optical probe and the vascular system, or a sample taken from the vascular system, using the calibrated fluorescent detector to obtain a spectrometric data set comprising a first emission fluorescent intensity curve from the first fluorescent tag and a second emission fluorescent intensity curve from the second fluorescent tag; (h) obtaining a raw ratio from the spectrometric data set to determine an apparent hematocrit of the subject from the species specific hematocrit curve; (i) obtaining a correction factor for determining the hematocrit of the mammalian subject; and U) applying the correction factor to the apparent hematocrit to determine the hematocrit of the mammalian subject. Other biometric indicators such as blood volume and volume of distribution, can also be subsequently determined.

The injectate may be introduced into the vascular system via bolus injection or by infusion.

An Oral Probe for Non-invasive Monitoring of fluorescence intensities

A stabilized oral probe of the present invention may be used to non-invasively monitor the fluorescent intensities of the fluorescent markers in the vascular system within the oral cavity of a mammalian subject. In an embodiment, the probe is placed under the tongue within the oral cavity.

The probe may comprise a longitudinal optical conduit having a proximal end and a distal end, wherein the distal end of the optical conduit forms a non-invasive interface between the optical conduit and the vascular system, and an oral stabilizing guide; wherein the fluorescent intensity of a fluorescent molecule in the vascular system is transmitted from the vascular system to the optical conduit through the optical interface at the distal end of the optical conduit to the proximal end of the optical conduit. The optical conduit may be a fiber optic cable. The proximal end of the optical conduit may be connected to a fluorescence detector to monitor the fluorescent intensities of the fluorescent markers in the vascular system. The optical conduit may transcend the oral stabilizing guide, or maybe set in mechanical communication with the surface of the stabilizing guide such that the oral stabilizing guide limits the movement of the optical conduit. The oral stabilizing guide may comprise a dental inset. An oral stabilizing guide may also contain an optical guide protrusion for maintaining position of an optical conduit under the tongue.

The oral probe of the present invention may further comprise a sterile sheath, which may be comprised of a uniform transparent material, or may be comprised of a transparent region and a moveable region. The oral probe may further comprise (a) a fitted region for maintaining a transparent sterile barrier between the optical interface and the tissue portion, and (b) a movable region for maintaining a sterile barrier between the optical positioning guide and the biological environment.

EXAMPLES

Example 1: Generation of calibration curves

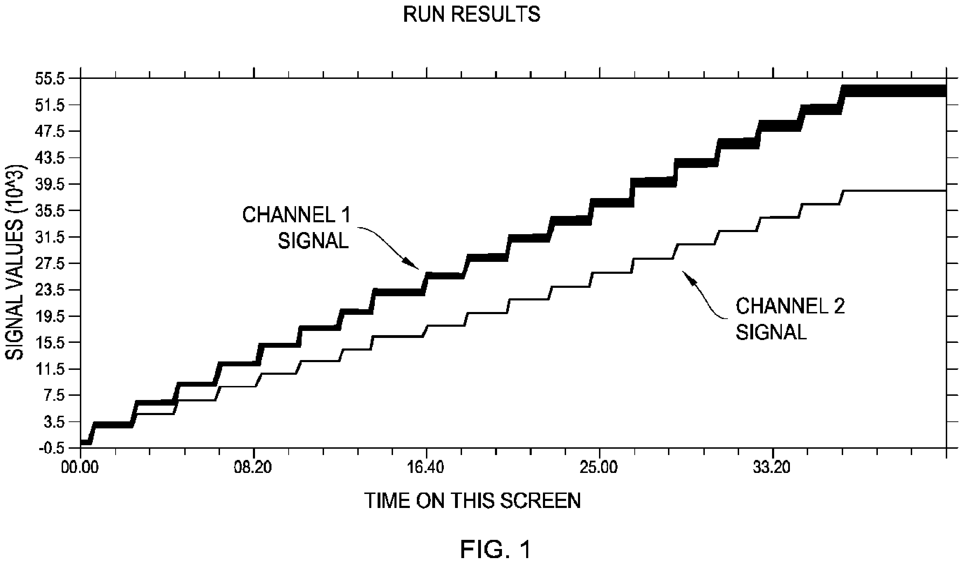

1. A step dose blood test set is run on a whole blood sample containing two fluorescent markers each having its distinct emission wavelength. An example of the results is shown in FIG. 1 with the upper curve representing the first emission signals from the first fluorescent marker or tag recorded in Channel 1 as the Channel 1 signal, and the second emission signals from the second fluorescent marker or tag recorded in Channel 2 as the Channel 2 signal. As discussed previously, this step dose blood test set can also be generated using one static marker having two fluorescent tags each tag having its distinct emission wavelength. Each fluorescent marker or each fluorescent tag may be referred to as a "fluorescent component" hereafter.

2. The average signal level of the "flat" or stable portion at each dose step for each fluorescent component is calculated.

3. Based on the known volume of blood ($V_t$) used, the known dose of VFI ($V_D$) and the known concentration of each VFI fluorescent component ($D_1$ or $D_2$) in the dose; the amount of each fluorescent marker present in the blood is calculated at each dose step (1).

$$x_{1;2} = V_D[D_{1;2}] \qquad (1)$$

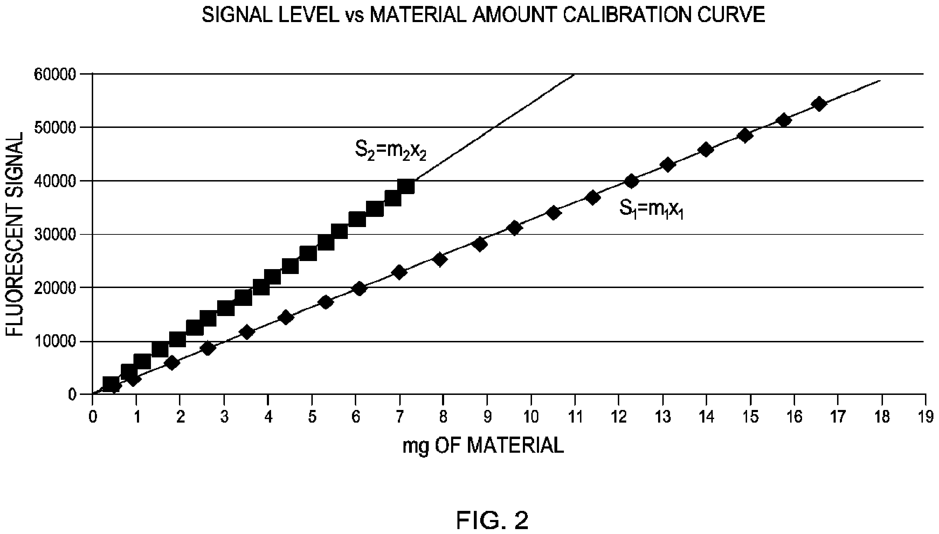

4. A fit line for the plot of each fluorescent component (intercept forced to zero) is generated, using the average signal level and amount of each component at each dose step calculated previously. The plot is shown in FIG. 2.

$$S_1 = m_1 x_1 \qquad (2)$$

$$S_2 = m_2 x_2 \qquad (3)$$

Where S is the signal level, m is the slope of the fit line and x is the amount (mg) of the material.

Example 2: Generation of a species specific hematocrit (HCT) calibration curve

1. A blood test is run with the single dose approach. With a known volume of blood ($V_t$) and a known HCT of the blood ($H_{calib}$), the volume of saline ($V_S$) needed for the test is calculated.

$$V_t - V_t H_{calib} = V_S \qquad (4)$$

2. The blood and the saline are equivalently dosed from the same VFI vial.

3. A predetermined volume of blood is removed from the test set and discarded. The same volume of dosed saline, as the blood previously removed, is injected back into the test set. This exchange will maintain the concentration of each component as well as the total volume of the test set, but alter the volume of distribution to HCT ratio. This step is repeated numerous times to generate multiple data points at which the volume of distribution and HCT ratio are different.

4. Each new point is allowed to stabilize before a new point is generated. A new HCT is calculated at each stable point.

$$\frac{(V_t - V_e)(H^0)}{V_t} = H'  \qquad (5)$$

Where $V_t$ is the total volume in the apparatus, $V_e$ is the volume exchanged (blood for saline), $H^0$ is the starting HCT (prior to volume exchange), and H' is the new HCT (post volume exchange).

5. The average signal level of a "flat" stable portion of data is taken at each HCT level generated during the test.

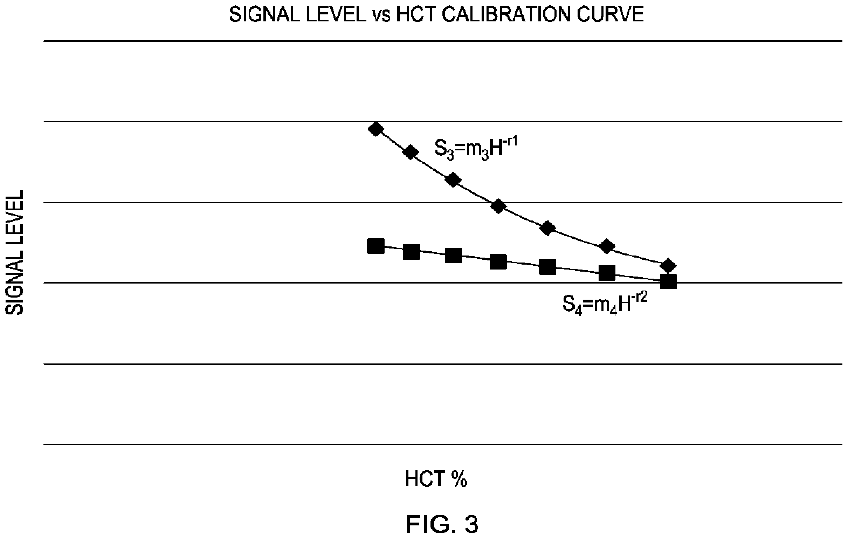

6. A plot of signal level vs. HCT is generated using the values calculated previously, as shown in FIG. 3.

7. A fit line is generated for each of the individual component plots. The equations generated will be in the form:

$$S_3 = m_3 H^{r1}  \qquad (6)$$

$$S_4 = m_4 H^{r2}  \qquad (7)$$

Where S is the signal level, H is the HCT, m is the slope, and r is a rate.

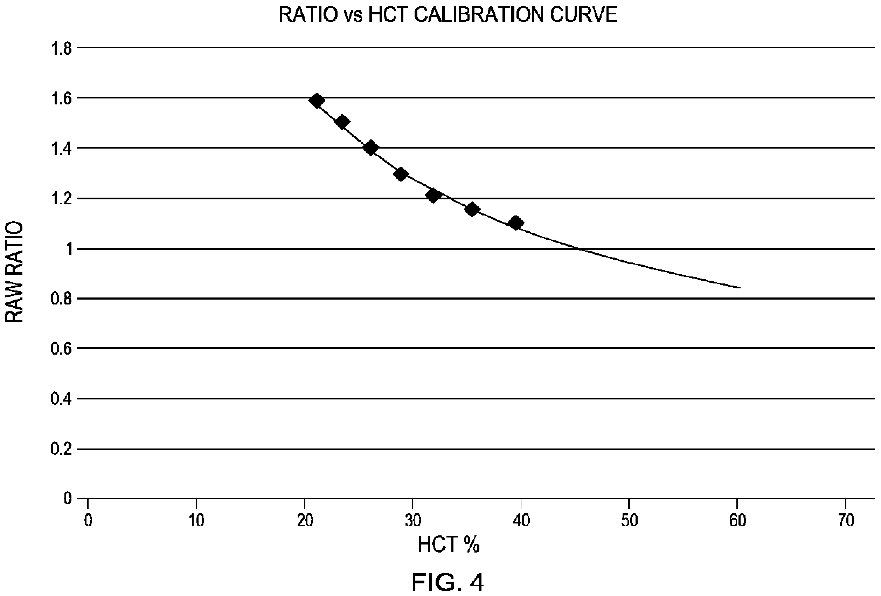

8. A fit line from the same HCT data taking the ratio of the signal levels of Component 1 to Component 2 (8), and plotting that ratio versus the HCT calculated at each stage in equation 5 is generated, as shown in FIG. 4.

$$R = S_3/S_4  \qquad (8)$$

The equation generated should take the form:

$$R = KH^{-q}  \qquad (9)$$

Where R is the ratio, K is a slope, H is the HCT and q is a rate.

Example 3: Determining various biometric indicators

When a test is run on a subject, the "batch" of VFI must be known because the signal calibration and HCT calibration curves used for interpretation must be based on the same "batch" of VFI given to the subject.

1. From a test data sample of FIG. 5, the raw ratio at $T_0$ ($R_{T0}$) and the average stable Component 2 (FD003) signal level ($S_{avg}$) are extracted. The lower curve in FIG. 5 represents Channel 1 signals, and the upper curve represents Channel 2 signals.

2. Using the raw ratio at $T_0$ ($R_{T0}$), the apparent HCT of the subject is calculated from the Ratio vs HCT Calibration Curve.

$$R_{T0} = KH_{-q}  \qquad (10)$$

$$H = H_{app}  \qquad (11)$$

3. Using the calculated apparent HCT and the Signal Level vs. Material Amount Calibration Curve; the amount of correction, C, is calculated and applied to the average signal level component.
From Equation 7:

$$S_{calib} = m_4 H_{calib}^{-r}  \qquad (12)$$

$$S_{app} = m_4 H_{app}^{-r}  \qquad (13)$$

$$If\ H_{app} < H_{calib}\ then\ S_{calib}/S_{app}$$

$$If\ H_{app} > H_{calib}\ then\ S_{app}/S_{calib}$$

$$S_{calib}/S_{app} = C  \qquad (14)$$

4. The correction factor, C, calculated in (14), is applied to the average signal level of component 2, $S_{avg}$, from the test data.

$$C * S_{avg} = S_C  \qquad (15)$$

5. The corrected signal, Sc , is used in equation (16) to determine the equivalent amount of material of component 2 based on the Signal Level vs. Material Amount Calibration Curve.

$$S_C = m_2 x  \qquad (16)$$

$$x = x_{eq}  \qquad (17)$$

6. From a ratio of the known amount (mg) of VFI component 2 dosed in the subject, $X_{sub}$, to a known volume used in calibration, $V_{distcalib}$, and a calculated equivalent amount of component 2 (mg), $x_{eq}$, to a volume of subject's $V_{distcalib}$, the subject's volume of distribution is calculated.

$$V_{distcalib} = V_t - V_t H_{calib} \qquad (18)$$

$$x_{eq}/V_{distcalib} = x_{sub}/V_{distsub} \qquad (19)$$

$$V_{distsub} = x_{sub}V_{distcalib}/x_{eq} \qquad (20)$$

7. The subject's HCT from the apparent HCT and the HCT offset are calculated.

$$H_{sub} = H_{app} + H_{os} \qquad (21)$$

8. The blood volume from the volume of distribution of the subject and the calculated subject HCT is calculated.

$$BV = V_{distsub}/H_{sub} \qquad (22)$$

Example 4: Example Calculation

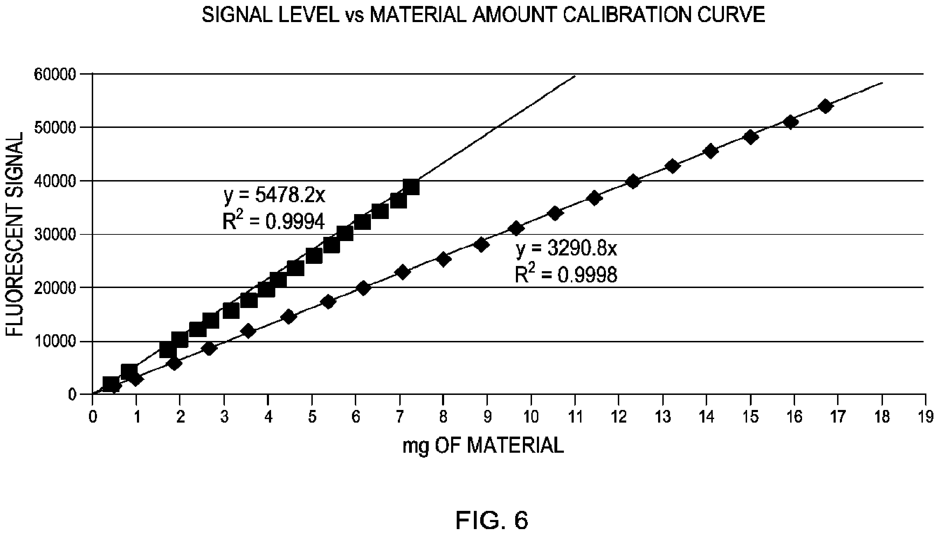

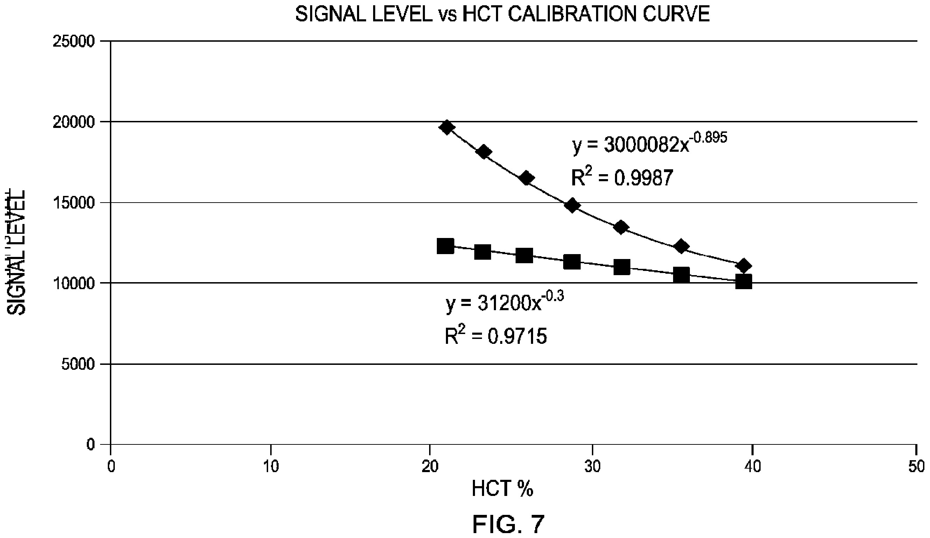

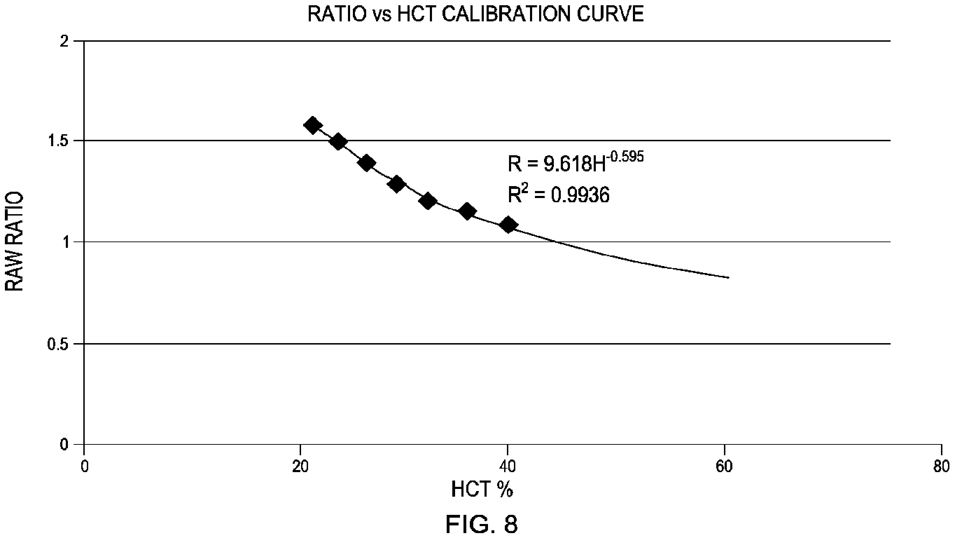

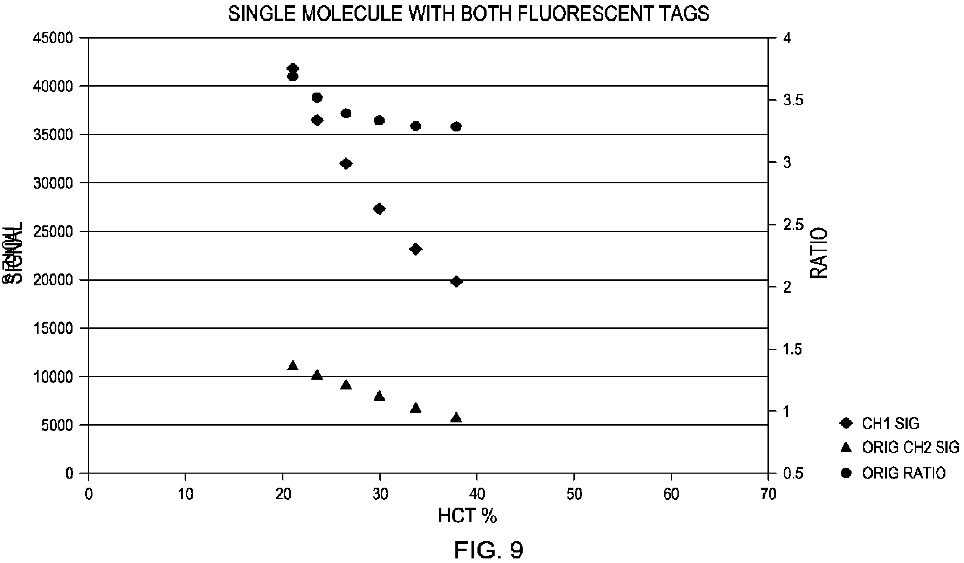

Calibration curves used in this example are shown in FIGS. 6 to 8. FIG. 9 is a calibration curve using one single static marker having two fluorescent tags.

For this example we will use the following set of known parameters:

  VFI dose concentration: 35mg/mL of Component 1 and 15mg/mL of Component 2
  Dose Volume: 3.0mL
  To Raw ratio: 1.2
  Avg Stable Component 2 Signal Level: 12000
  Calibration curve's test volume: 100mL
  Calibration curve's test HCT: 38%

1. From the raw ratio at $T_0$, $R_{T0}$, the apparent HCT of the subject is calculated from the Ratio vs HCT Calibration Curve.

$$1.2 = 9.618H^{-0.595} \qquad (23)$$

$$H_{app} = 33\% \qquad (24)$$

2. Using the calculated apparent HCT and the Signal Level vs HCT Calibration Curve; the amount of correction, C, needed to be applied to the average signal level of component 2 ($S_{avg}$), is calculated using the following (25, 26,27).

$$S_{calib} = 31200(38)^{-0.3} \qquad (25)$$
$$S_{calib} = 10476$$

$$S_{app} = 31200(33)^{-0.3} \qquad (26)$$
$$S_{app} = 10930$$

$H_{app}{<}H_{calib}$ so $S_{calib}/S_{app}$:
$$10476/10930 = 0.958 = C \qquad (27)$$

3. The correction factor, C, is applied to the average signal level of component 2, $S_{avg}$, from the test data.

$$(0.958)(12000) = S_C \qquad (28)$$
$$S_C = 11496$$

4. The corrected signal, $S_C$, in equation (16) is used to determine the equivalent amount of material of component 2 based on the Signal Level vs Material Amount Calibration Curve.

$$11496 = 5478.2x \qquad (29)$$

$$x = 2.09\,\mathrm{mg} = x_{eq} \qquad (30)$$

5. From a ratio of the known amount (mg) of VFI component 2 dosed in the subject, $X_{sub}$, and a known volume used in calibration, $V_{distcalib}$, to a calculated equivalent amount of component 2 (mg), $x_{eq}$, and the volume of distribution of the subject, $V_{distcalib}$, the subject's volume of distribution is calculated.

$$V_{distcalib} = V_t - V_t H_{calib} \qquad (31)$$

$$V_{distcalib} = 100 - 100 * (.38) \qquad (32)$$

$$2.07/62 = (3 * 15)/V_{distsub}$$

$$V_{distsub} = 1334.9\mathrm{mL} \qquad (33)$$

6. The subject's HCT is calculated from the apparent HCT and the HCT offset.

$$H_{sub} = 33 + 5 = 38\% \qquad (34)$$

7. Blood volume is calculated from the volume of distribution of the subject and the calculated subject HCT.

$$BV = 1334.9/0.38 \qquad (35)$$

$$BV = 3513\text{mL}$$

While the invention has been illustrated and described in detail in the drawings and foregoing description, the same is to be considered as illustrative and not restrictive in character. It is understood that the embodiments have been shown and described in the foregoing specification in satisfaction of the best mode and enablement requirements. It is understood that one of ordinary skill in the art could readily make insubstantial changes and modifications to the above-described embodiments and that it would be impractical to attempt to describe all such embodiment variations in the present specification. Accordingly, it is understood that all changes and modifications that come within the spirit of the invention are desired to be protected.

While the specific embodiments have been illustrated and described, numerous modifications come to mind without significantly departing from the spirit of the invention, and the scope of protection is only limited by the scope of the accompanying Claims.

**Additional aspects of the disclosure are presented in the following numbered clauses:**

1. An injectate for measuring hematocrit of a mammalian subject comprising:

(a) a first fluorescent marker having a first excitation wavelength and a first emission wavelength;
(b) a second fluorescent marker having a second excitation wavelength and a second emission wavelength; and
(c) an injectate carrier;

wherein the first fluorescent marker is a dynamic molecule and the second fluorescent marker is a static molecule; and
wherein the injectate has undergone calibration to provide a calibration identification containing parameters of the injectate.

2. The injectate of clause 1 wherein the parameters of the calibration identification comprises a first fluorescent intensity of the first fluorescent marker and a second fluorescent intensity of the second fluorescent marker.

3. The injectate of clause 2 wherein the parameters of the calibration identification further comprises a lot number of the injectate.

4. The injectate of clause 1 wherein the parameters of the injectate are captured in a digital format.

5. The injectate of clause 1 wherein the parameters of the injectate are communicated to a fluorescent detector to calibrate the fluorescent detector.

6. The injectate of clause 1 wherein the mammal is a human.

7. A method for measuring hematocrit of a mammalian subject comprising:

(a) providing an injectate comprising:

(i) a first fluorescent marker having a first excitation wavelength and a first emission wavelength;
(ii) a second fluorescent marker having a second excitation wavelength and a second emission wavelength; and
(iii) an injectate carrier;

wherein the first fluorescent marker is associated with a dynamic molecule, and the second fluorescent marker is associated with a static molecule;
(b) calibrating the injectate to obtain a calibration identification of the injectate containing parameters of the injectate;
(c) inputting the parameters of the calibration identification of the injectate to a fluorescent detector to calibrate the fluorescent detector;
(d) obtaining a species specific hematocrit curve;
(e) introducing the injectate into the vascular system of the mammalian subject;
(f) exciting the first fluorescent marker with a first excitation wavelength and exciting the second fluorescent marker with a second excitation wavelength at an optical interface of an optical probe and the vascular system, or a sample taken from the vascular system;
(g) measuring the first emission intensity of the first emission wavelength of the first fluorescent marker and measuring the second emission intensity of the second fluorescent marker at the optical interface of the optical probe and the vascular system, or a sample taken from the vascular system, using the calibrated fluorescent detector to obtain a spectrometric data set comprising a first emission fluorescent intensity curve from the first fluorescent marker and a second emission fluorescent intensity curve from the second fluorescent marker;

(h) obtaining a ratio of the first fluorescent marker to the second fluorescent marker at $T_0$ from the spectrometric data set to determine an apparent hematocrit of the subject from the species specific hematocrit curve;

(i) obtaining a correction factor for determining the hematocrit of the mammalian subject; and

(j) applying the correction factor to the apparent hematocrit to determine the hematocrit of the mammalian subject.

8. The method of clause 7 wherein the hematocrit is determined by a calibrated spectrometric analyzer comprising an input for a spectrometric data set, an input for a calibration identification, a computational engine for calculating hematocrit and an output for reporting a calculated hematocrit.

9. The method of clause 7 further comprising calculating other biometric indicators of the mammalian subject.

10. The method of clause 9 wherein the biometric indicator is blood volume.

11. The method of clause 9 wherein the biometric indicator is volume of distribution.

12. The method of clause 9 wherein the biometric indicator is glomerular filtration rate.

13. The method of clause 7 wherein the mammal is a human.

14. An injectate for measuring hematocrit of a mammalian subject comprising:

(a) a static marker having a first fluorescent tag and a second fluorescent tag, wherein the first fluorescent tag has a first excitation wavelength and a first emission wavelength, and the second fluorescent tag has a second excitation wavelength and a second emission wavelength; and

(b) an injectate carrier;

wherein the injectate has undergone calibration to provide a calibration identification containing parameters of the injectate.

15. The injectate of clause 14 wherein the parameters of the calibration identification comprises a first fluorescent intensity of the first fluorescent tag and a second fluorescent intensity of the second fluorescent tag.

16. The injectate of clause 15 wherein the parameters of the calibration identification further comprises a lot number of the injectate.

17. The injectate of clause 14 wherein the parameters of the injectate are captured in a digital format.

18. The injectate of clause 14 wherein the parameters of the injectate are communicated to a fluorescent detector to calibrate the fluorescent detector.

19. The injectate of clause 14 wherein the mammal is a human.

20. A method for measuring hematocrit of a mammalian subject comprising:

(a) providing an injectate comprising:

(i) a static marker having a first fluorescent tag and a second fluorescent tag, wherein the first fluorescent tag having a first excitation wavelength and a first emission wavelength and the second fluorescent tag having a second excitation wavelength and a second emission wavelength; and

(ii) an injectate carrier;

(b) calibrating the injectate to obtain a calibration identification of the injectate containing parameters of the injectate;

(c) inputting the parameters of the calibration identification of the injectate to a fluorescent detector to calibrate the fluorescent detector;

(d) obtaining a species specific hematocrit curve;

(e) introducing the injectate into vascular system of the mammalian subject;

(f) exciting the first fluorescent tag with a first excitation wavelength and exciting the second fluorescent tag with a second excitation wavelength at an optical interface of an optical probe and the vascular system, or a sample taken from the vascular system;

(g) measuring the first emission intensity of the first emission wavelength of the first fluorescent tag and measuring the second intensity of the second emission wavelength of the second fluorescent tag at the optical interface of the optical probe and the vascular system, or at a sample taken from the vascular system, using the calibrated fluorescent detector to obtain a spectrometric data set comprising a first emission fluorescent intensity curve from the first fluorescent tag and a second emission fluorescent intensity curve from the second fluorescent tag;

(h) obtaining a ratio of the first fluorescent tag to the second fluorescent tag at $T_0$ from the spectrometric data set to determine an apparent hematocrit of the subject from the spectrometric data set to determine an apparent hematocrit of the subject from the species specific hematocrit curve;

(i) obtaining a correction factor for determining the hematocrit of the mammalian subject; and

(j) applying the correction factor to the apparent hematocrit to determine the hematocrit of the mammalian subject.

21. The method of clause 20 wherein the hematocrit is determined by a calibrated spectrometric analyzer comprising an input for a spectrometric data set, an input for a calibration identification, a computational engine for calculating hematocrit and an output for reporting a calculated hematocrit.

22. The method of clause 20 further comprising calculating other biometric indicators of the mammalian subject.

23. The method of clause 22 wherein the biometric indicator is blood volume.

24. The method of clause 22 wherein the biometric indicator is volume of distribution.

25. The method of clause 20 wherein the mammal is a human.

26. An oral probe for use within oral cavity of a mammalian subject for measuring fluorescent intensity of a fluorescent molecule in vascular system of the mammalian subject comprising:

(a) a longitudinal optical conduit having a proximal end and a distal end;
(b) an optical interface at the distal end of the optical conduit; and
(c) an oral stabilizing guide to limit movement of the optical conduit within the oral cavity;

wherein fluorescent intensity of a fluorescent molecule in the vascular system is transmitted from the vascular system to the optical conduit through the optical interface at the distal end of the optical conduit to the proximal end of the optical conduit.

27. The oral probe of clause 26 wherein the optical conduit is a fiber optic cable.

28. The oral probe of clause 26 wherein the probe is placed under the tongue of the mammalian subject within the oral cavity.

29. The oral probe of clause 26 wherein the proximal end of the optical conduit is connected to a fluorescence detector.

30. The oral probe of clause 26 wherein the probe further comprises a sterile sheath along the optical conduit.

31. A method for measuring the glomerular filtration rate (GFR) of a mammalian subject comprising:

(a) providing an injectate comprising:

(i) a first fluorescent marker having a first excitation wavelength and a first emission wavelength;
(ii) a second fluorescent marker having a second excitation wavelength and a second emission wavelength; and
(iii) an injectate carrier;

wherein the first fluorescent marker is associated with a dynamic molecule, and the second fluorescent marker is associated with a static molecule;

(b) introducing the injectate into the vascular system of the mammalian subject;
(c) exciting the first fluorescent marker with a first excitation wavelength and exciting the second fluorescent marker with a second excitation wavelength at an optical interface of an optical probe and a sample taken from the vascular system at periodic intervals;
(d) measuring the first emission intensity of the first emission wavelength of the first fluorescent marker and the second emission intensity of the second fluorescent marker of the sample using a fluorescent detector to obtain a spectrometric data set comprising a measured ratio of the concentration of the first fluorescent marker to the second fluorescent marker at 10 to 60 minute intervals, and a predicted concentration of the first fluorescent marker at $T_0$, and subsequent data points taken from samples over a time period of 1 to 2 hours to determine the GFR for the mammal.

32. The method of clause 31 wherein the mammal is a human.

33. The method of clause 31 wherein the samples are taken over intervals of 10 to 15 minutes.

**Claims**

1. An oral probe for use within oral cavity of a mammalian subject for measuring fluorescent intensity of a fluorescent molecule in vascular system of the mammalian subject comprising:

(a) a longitudinal optical conduit having a proximal end and a distal end;
(b) an optical interface at the distal end of the optical conduit; and
(c) an oral stabilizing guide to limit movement of the optical conduit within the oral cavity;

wherein fluorescent intensity of a fluorescent molecule in the vascular system is transmitted from the vascular system to the optical conduit through the optical interface at the distal end of the optical conduit to the proximal end of the optical conduit.

2.  The oral probe of Claim 1 wherein the optical conduit is a fiber optic cable.

3.  The oral probe of Claim 1 or Claim 2 wherein the probe is placed under the tongue of the mammalian subject within the oral cavity.

4.  The oral probe of any preceding Claim wherein the proximal end of the optical conduit is connected to a fluorescence detector.

5.  The oral probe of any preceding Claim wherein the probe further comprises a sterile sheath along the optical conduit.

RUN RESULTS

FIG. 1

EP 3 207 873 A1

SIGNAL LEVEL vs MATERIAL AMOUNT CALIBRATION CURVE

FIG. 2

SIGNAL LEVEL vs HCT CALIBRATION CURVE

$S_3 = m_3 H^{-r1}$

$S_4 = m_4 H^{-r2}$

SIGNAL LEVEL

HCT %

FIG. 3

RATIO vs HCT CALIBRATION CURVE

HCT %

FIG. 4

RAW RATIO

RUN RESULTS

FIG. 5

EP 3 207 873 A1

SIGNAL LEVEL vs MATERIAL AMOUNT CALIBRATION CURVE

$y = 5478.2x$
$R^2 = 0.9994$

$y = 3290.8x$
$R^2 = 0.9998$

FLUORESCENT SIGNAL

mg OF MATERIAL

FIG. 6

EP 3 207 873 A1

SIGNAL LEVEL vs HCT CALIBRATION CURVE

$y = 3000082x^{-0.895}$

$R^2 = 0.9987$

$y = 31200x^{-0.3}$

$R^2 = 0.9715$

SIGNAL LEVEL

HCT %

FIG. 7

EP 3 207 873 A1

RATIO vs HCT CALIBRATION CURVE

$R = 9.618H^{-0.595}$

$R^2 = 0.9936$

FIG. 8

SINGLE MOLECULE WITH BOTH FLUORESCENT TAGS

◆ CH1 SIG
▲ ORIG CH2 SIG
● ORIG RATIO

RATIO

SIGNAL

HCT %

FIG. 9

Europäisches
Patentamt

European
Patent Office

Office européen
des brevets

**EUROPEAN SEARCH REPORT**

Application Number

EP 17 16 4592

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 7 386 333 B1 (BIRECKI HENRYK [US] ET AL) 10 June 2008 (2008-06-10)<br>* column 3, lines 38-41 *<br>* column 5, lines 22-49 *<br>* figures 1, 2a *<br>----- | 1-4 | INV.<br>A61B6/00<br>A61B5/20<br>A61B5/00 |
| X | WO 2012/007769 A1 (LUX INNOVATE LTD [GB]; PERFECT EMMA; LONGBOTTOM CHRIS; HAUGHEY ANNE-MA) 19 January 2012 (2012-01-19)<br>* page 1, lines 4-12 *<br>* page 3, lines 17-18 *<br>* page 5, lines 21-25 *<br>* page 11, line 10 - page 12, line 6 *<br>* page 13, line 16 - page 14, line 7 *<br>* page 14, lines 13-16 *<br>* figures 1, 2, 3 *<br>----- | 1-5 | |
| X | US 2008/082000 A1 (THOMS MICHAEL [DE]) 3 April 2008 (2008-04-03)<br>* paragraphs [0005], [0039] - [0041], [0078], [0079], [0086] - [0088] *<br>* figures 1-5 *<br>----- | 1-3 | TECHNICAL FIELDS SEARCHED (IPC)<br>A61B<br>A61K |
| X | US 2011/318712 A1 (REDDY MEGHA [US] ET AL) 29 December 2011 (2011-12-29)<br>* paragraphs [0025] - [0030] *<br>* figures 1, 2, 5 *<br>----- | 1-4 | |
| X | US 6 485 300 B1 (MULLER WERNER [DE] ET AL) 26 November 2002 (2002-11-26)<br>* column 8, line 19 - column 9, line 7 *<br>* column 13, line 55 - column 14, line 6 *<br>* figures 7, 8 *<br>----- | 1-4 | |

The present search report has been drawn up for all claims

| Place of search | Date of completion of the search | Examiner |
|---|---|---|
| The Hague | 5 July 2017 | Trattner, Barbara |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 17 16 4592

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2017

| Patent document cited in search report | | Publication date | Patent family member(s) | | Publication date |
|---|---|---|---|---|---|
| US 7386333 | B1 | 10-06-2008 | NONE | | |
| WO 2012007769 | A1 | 19-01-2012 | AU | 2011278051 A1 | 31-01-2013 |
| | | | BR | 112013001060 A2 | 24-05-2016 |
| | | | CA | 2805160 A1 | 19-01-2012 |
| | | | CN | 103153166 A | 12-06-2013 |
| | | | EP | 2592993 A1 | 22-05-2013 |
| | | | JP | 5878529 B2 | 08-03-2016 |
| | | | JP | 2013531546 A | 08-08-2013 |
| | | | US | 2013189641 A1 | 25-07-2013 |
| | | | WO | 2012007769 A1 | 19-01-2012 |
| US 2008082000 | A1 | 03-04-2008 | CN | 1972626 A | 30-05-2007 |
| | | | DE | 102004024494 A1 | 08-12-2005 |
| | | | DK | 1750574 T3 | 23-02-2015 |
| | | | EA | 200602018 A1 | 31-08-2007 |
| | | | EP | 1750574 A1 | 14-02-2007 |
| | | | EP | 2338410 A1 | 29-06-2011 |
| | | | EP | 2363060 A1 | 07-09-2011 |
| | | | ES | 2530394 T3 | 02-03-2015 |
| | | | HK | 1106687 A1 | 31-12-2009 |
| | | | JP | 5026962 B2 | 19-09-2012 |
| | | | JP | 2007537802 A | 27-12-2007 |
| | | | JP | 2012016601 A | 26-01-2012 |
| | | | US | 2008082000 A1 | 03-04-2008 |
| | | | WO | 2005110206 A1 | 24-11-2005 |
| US 2011318712 | A1 | 29-12-2011 | AU | 2011276691 A1 | 10-01-2013 |
| | | | BR | 112012033368 A2 | 29-11-2016 |
| | | | CA | 2802711 A1 | 12-01-2012 |
| | | | EP | 2588000 A1 | 08-05-2013 |
| | | | JP | 5992405 B2 | 14-09-2016 |
| | | | JP | 2013531534 A | 08-08-2013 |
| | | | RU | 2013103712 A | 10-08-2014 |
| | | | US | 2011318712 A1 | 29-12-2011 |
| | | | WO | 2012005891 A1 | 12-01-2012 |
| US 6485300 | B1 | 26-11-2002 | AR | 015788 A1 | 16-05-2001 |
| | | | AT | 270522 T | 15-07-2004 |
| | | | AU | 750410 B2 | 18-07-2002 |
| | | | BR | 9910509 A | 02-01-2001 |
| | | | CA | 2333226 A1 | 25-11-1999 |
| | | | CN | 1309545 A | 22-08-2001 |
| | | | CZ | 20004296 A3 | 16-01-2002 |
| | | | DE | 69918549 D1 | 12-08-2004 |
| | | | DE | 69918549 T2 | 28-07-2005 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 17 16 4592

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2017

| Patent document cited in search report | Publication date | Patent family member(s) | Publication date |
|---|---|---|---|
| | | EA 200001075 A1 | 27-08-2001 |
| | | EP 1079731 A1 | 07-03-2001 |
| | | ES 2224706 T3 | 01-03-2005 |
| | | HU 0102865 A2 | 28-12-2001 |
| | | JP 2002515276 A | 28-05-2002 |
| | | MX PA00011274 A | 06-09-2004 |
| | | PE 06222000 A1 | 23-07-2000 |
| | | PL 344439 A1 | 05-11-2001 |
| | | TW 412405 B | 21-11-2000 |
| | | US 6485300 B1 | 26-11-2002 |
| | | WO 9959462 A1 | 25-11-1999 |
| | | ZA 200006676 B | 21-08-2001 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61600182 A
- US 12425827 B
- US 12946471 B
- US 2009040994 PCT
- US 1032997 W